# EUROPÄISCHE PATENTSCHRIFT

(11) **EP 0 794 952 B1**
(45) Veröffentlichungstag und Bekanntmachung des Hinweises auf die Patenterteilung: **23.02.2000**
(21) Anmeldenummer: 96925683.3
(22) Anmeldetag: 08.07.1996
(51) Int. Cl.: C07D 317/36, A61K 6/00

(54) **POLYMERISIERBARE AROMATISCHE CARBONSÄUREN UND CARBONSÄUREANHYDRIDE MIT CYCLISCHEN CARBONATGRUPPEN SOWIE ZUBEREITUNGEN, ENTHALTEND DIESE VERBINDUNGEN**
POLYMERISABLE AROMATIC CARBOXYLIC ACID AND CARBOXYLIC ACID ANHYDRIDES WITH CYCLIC CARBONATE GROUPS AND COMPOSITIONS THAT CONTAIN THESE COMPOUNDS
ACIDES CARBOXYLIQUES AROMATIQUES ET ANHYDRIDES D'ACIDES CARBOXYLIQUES POLYMERISABLES, COMPRENANT DES GROUPES CARBONATES CYCLIQUES ET COMPOSITIONS CONTENANT CES COMPOSES

(30) Priorität: 10.07.1995 DE 19525033
(43) Veröffentlichungstag der Anmeldung: 17.09.1997
(73) Patentinhaber: Heraeus Kulzer GmbH & Co.KG, 63450 Hanau (DE)
(72) Erfinder: PODSZUN, Wolfgang, D-51061 Köln (DE); HEILIGER, Ludger, D-51373 Leverkusen (DE); FINGER, Werner, D-41469 Neuss (DE); GRUNWALD, Martin, D-50259 Pulheim (DE); CASSER, Carl, D-51061 Köln (DE)
(74) Vertreter: Kühn, Hans-Christian
(86) Internationale Anmeldenummer: EP9602987
(87) Internationale Veröffentlichungsnummer: WO9703063

(56) Entgegenhaltungen:
- EP-A- 0 471 252
- DE-A- 4 324 614
- US-A- 4 148 988
- DENTAL MATERIALS JOURNAL, Bd. 4, Nr. 1, 1985, Seiten 33-39, XP000569813 TADAO FUKUSHIMA ET AL.: "Application of Functional Monomers for Dental Use (Part-9) Syntheses of Succinoxy Methacrylates and Their Adhesion to Polished and Etched Tooth Surfaces"

## Beschreibung

Die Erfindung betrifft neue polymerisierbare aromatische Carbonsäuren und Carbonsäureanhydride mit cyclischen Carbonatgruppen sowie Zubereitungen dieser Verbindungen, vorzugsweise zur Verwendung in der Dentaltechnik.

Ein besonders gravierendes Problem in der konservierenden Zahnheilkunde besteht in der dauerhaften, randspaltfreien Verklebung von Kunststoff-Füllungsmaterialien mit der Zahnhartsubstanz (Dentin und Schmelz). Im Dentalbereich werden aushärtbare Materialien als Füllungsmaterialien bei Zahnreparaturen verwendet. Als aushärtbare Materialien werden im allgemeinen Füllungsmaterialien auf Acrylatbasis bevorzugt, die durch radikalische Polymerisation ausgehärtet werden. Ein Nachteil dieser Materialien besteht darin, daß sie während der Aushärtung schrumpfen und so zur Randspaltbildung beitragen. Die Kunststoff-Füllungen haben den zusätzlichen Nachteil, daß sie schlecht am Dentin haften bleiben.

Um die Anbindung an die Zahnhartsubstanz zu verbessern, kann man sogenannte Adhäsive oder Haftvermittler einsetzen. Als wirksamer Bestandteil solcher Adhäsive für Füllungen im Dentalbereich werden z. B. Methacryloyloxyalkylderivate aromatischer Carbonsäuren eingesetzt. So werden beispielsweise in US-A-4 148 988 Gemische von Trimellitsäure-4-methacryloyloxyethylester (4-MET) oder Trimellitsäureanhydrid-4-methacryloyloxyethylester (4-META) mit ethylenisch ungesättigten Monomeren und Polymerisationsinitiatoren beschrieben.

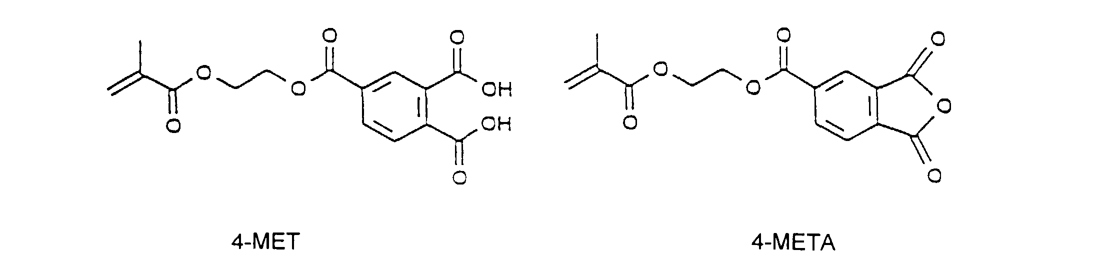

Ein sich aus 4-META aufbauendes Handelsprodukt (Superbond von Sun Medical) muß zum Erhalt der applikationsfertigen Form mit Methylmethacrylat (MMA), Polymethylmethacrylat (PMMA) und teilweise oxidiertem Tri-n-butylboran (TBB) gemischt werden (MMA-4-META-TBB-Resin).

In EP 0 471 252 B1 werden N-Alkyl-N-(meth)acryloyloxyalkylcarboxamide aromatischer Carbonsäuren und Carbonsäureanhydride als Komponenten für Adhäsive vorgeschlagen. Für diese (Meth)acryloyloxyalkylderivate ergeben sich deutlich vereinfachte Applikationsformulierungen.

Aus DE-A-43 24 614 sind cyclische Carbonat-Gruppen aufweisende (Meth)Acrylsäureester mit hoher Photopolymerisationsfähigkeit bekannt. Die Ester - vorzugsweise aliphatische Ester - bilden zusammen mit Photopolymerisationsinitiatoren die lichtempfindliche Schicht von trocken verarbeitbarem Aufzeichnungsmaterial, das weitere Schichten, zum Beispiel eine Schicht zur Verbesserung der Haftung der lichtempfindlichen Schicht am Schichtträger, enthalten kann.

Ein Nachteil der bekannten (Meth)acryloyloxyalkylderivate aromatischer Carbonsäuren ist ihre relativ schlechte Polymerisationsfähigkeit. Dies bringt mehrere schwerwiegende Nachteile mit sich. Beispielsweise kann die Aushärtung unvollständig sein, können Restmonomere zurückbleiben und müssen drastische Bedingungen bei der Härtung, z.B. lange Belichtungszeiten, angewendet werden.

Es wurde nun gefunden, daß sich mit Hilfe der neuen polymerisierbaren aromatischen Carbonsäuren und Carbonsäureanhydride mit cyclischen Carbonatgruppen Adhäsive, vorzugsweise zur Behandlung der Zahnhartsubstanz geeignete, formulieren lassen, die eine deutlich verbesserte Polymerisationsfähigkeit aufweisen.

Die neuen Verbindungen entsprechen den in der Tabelle 1 angegebenen Formeln.

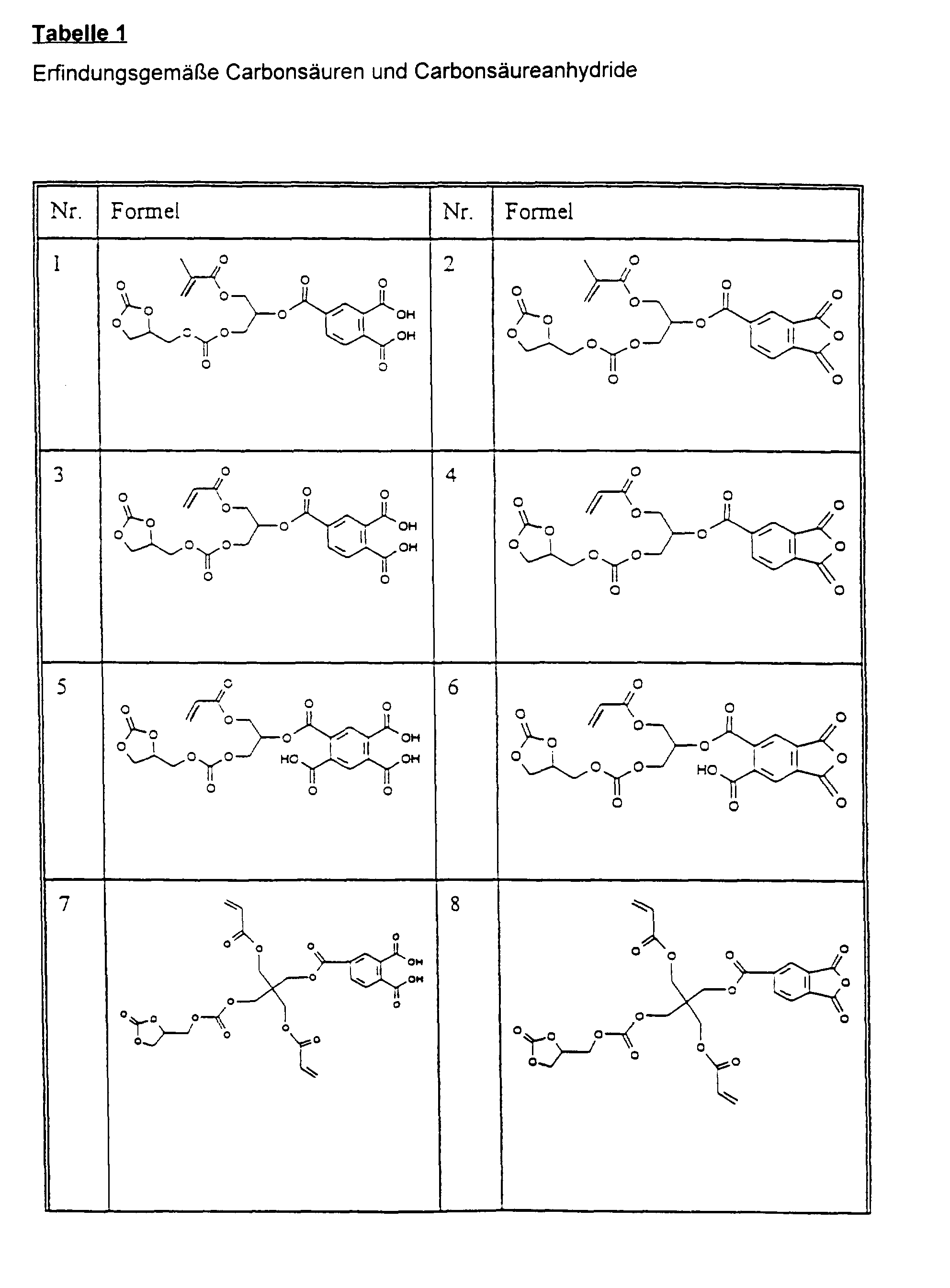

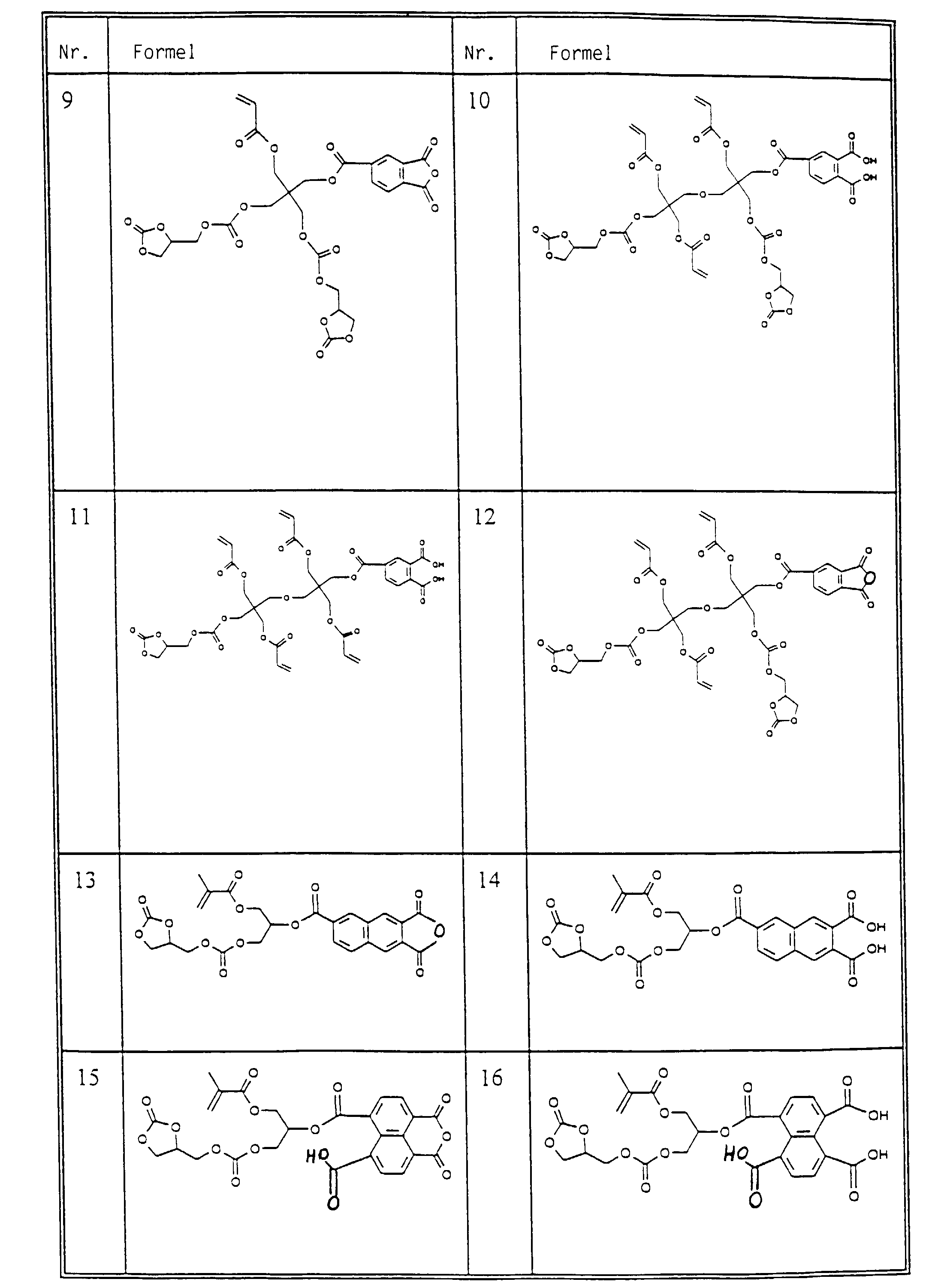

Die Herstellung der erfindungsgemäßen polymerisierbaren aromatischen Carbonsäuren und Carbonsäureanhydride erfolgt zweckmäßigerweise durch Umsetzung von aromatischen Monoanhydriden oder Dianhydriden mit im Alkohol-Rest durch Gruppen der Formel (I) substituierten Hydroxyalkyl(meth)acrylaten.

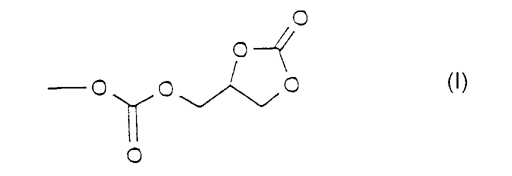

Als Monoanhydride werden die käuflichen Trimellitsäure-Derivate
1,2,4-Benzoltricarbonsäureanhydridchlorid und 1,2,4-Benzoltricarbonsäureanhydrid, die literaturbekannten Hemimellitsäure-Derivate 1,2,3-Benzoltricarbonsäureanhydrid und
1,2,3-Benzoltricarbonsäureanhydridchlorid und die Naphthalintricarbonsäure-Derivate 1,2,6-, und 1,4,5-Naphthalintricarbonsäureanhydridchlorid bevorzugt.

Als Dianhydride werden die käuflichen Verbindungen Benzol-1,2,4,5-tetracarbonsäuredianhydrid (Pyromellitsäuredianhydrid), Naphthalin-1,4,5,8-tetracarbonsäuredianhydrid und Naphthalin-2,3,6,7-tetracarbonsäuredianhydrid, welches sich einfach durch Dehydratisierung aus der bekannten Naphthalin-2,3,6,7-tetracarbonsäure bildet, bevorzugt.

Die den erfindungsgemäßen polymerisierbaren aromatischen Carbonsäuren und Carbonsäureanhydriden zugrunde liegenden Hydroxyalkyl(meth)acrylate sind durch schrittweise Veresterung von Polyhydroxyalkylverbindungen mit z. B. (Meth)acrylsäurechlorid und dem Chlorameisensäureester der Formel (II) zugänglich.

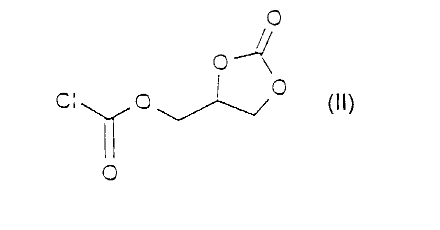

Der Chlorameisensäureester kann durch Phosgenierung von Glycerin hergestellt werden. Dieser Syntheseschritt wird in US 2 446 145 ausführlich beschrieben.

Die Herstellung der erfindungsgemäßen polymerisierbaren aromatischen Carbonsäuren und Carbonsäureanhydride erfolgt vorzugsweise in einem organischen Lösungsmittel. Geeignete organische Lösungsmittel sind aprotische Lösungsmittel, wie Dioxan, Tetrahydrofuran, N,N-Dimethylformamid, N,N-Dimethylacetamid, Dimethylsulfonamid und Aceton. Bevorzugt geeignet sind Toluol und Diethylether. Besonders bevorzugt sind Xylol, Methylenchlorid, Chloroform und Methyltert.-butylether.

Ein geeigneter Temperaturbereich zur Herstellung der erfindungsgemäßen polymerisierbaren aromatischen Carbonsäuren und Carbonsäureanhydride liegt zwischen -30 und 110° C. Bevorzugt wird die Reaktion zwischen -10 und 50° C und besonders bevorzugt zwischen -5 und 30° C durchgeführt. Bei der Herstellung können zusätzlich anorganische oder organische Basen verwendet werden.

Bevorzugte anorganische Basen sind die schwach basischen Carbonate und Hydrogencarbonate des Natriums und Kaliums. Bevorzugte organische Basen sind tert. Amine, wobei Triethylamin und Pyridin besonders bevorzugt sind. Die Basen werden, bezogen auf das Anhydrid, in einer äquimolaren bis 5-fach molaren Menge eingesetzt, wobei ein 2- bis 3-fach molarer Überschuß bevorzugt wird. Die organischen Basen wirken zusätzlich lösungsvermittelnd.

Zur Herstellung der erfindungsgemäßen polymerisierbaren aromatischen Carbonsäuren mit benachbarten Carbonsäuregruppen können zunächst die entsprechenden Anhydride synthetisiert werden. Aus den Anhydriden sind die Dicarbonsäuren durch Hydrolyse bei Temperaturen zwischen 5 und 100° C, bevorzugt zwischen 20 und 50° C, zugänglich. Die Hydrolyse kann nach Isolierung der Anhydride erfolgen. Die direkte Hydrolyse des Reaktionsansatzes ist aber auch möglich. Zur Durchführung der Hydrolyse wird Wasser in äquimolarer, bevorzugt jedoch in über 10-fach molarer Menge zugegeben. Die Hydrolyse kann durch gezielte Zugabe von Säuren, vor allem Schwefelsäure, Phosphorsäure, Toluolsulfonsäure oder sauren lonenaustauschern, oder durch Zugabe von Basen, wie Natrium- und Kaliumhydroxid, -carbonat oder -hydrogencarbonat, katalysiert werden.

Die Reaktionsfähigkeit von durch Polymerisation härtbaren Verbindungen kann sehr gut mit der Photo-DSC-Methode (Differential Scanning Calorimetry) charakterisiert werden.

Bei dieser Methode werden photoaktivierte Proben in einer DSC-Apparatur mit einer intensiven Strahlungsquelle, z. B. einer Halogenlampe mit Wärmeschutzfilter, bestrahlt. Der Wärmefluß wird unter Bestrahlung als Funktion der Zeit registriert. Als Referenz werden Proben gleicher Zusammensetzung ohne Photoinitiator eingesetzt. Als Maß für die Reaktionsgeschwindigkeit wird der Wert t-max ermittelt, t-max ist die Zeit von Bestrahlungsbeginn bis zum Erreichen des Maximums der Reaktion (maximaler Wärmefluß). Je kleiner t-max ist, um so größer ist die Photoreaktivität.

Die erfindungsgemäßen Zubereitungen enthalten außer den neuen polymerisierbaren aromatischen Carbonsäuren und Carbonsäureanhydriden Lösungsmittel, Initiatoren, Coaktivatoren und gegebenenfalls weitere (Meth)acrylsäureester als Comonomere. Es können auch Gemische verschiedener erfindungsgemäßer polymerisierbarer aromatischer Carbonsäuren und Carbonsäureanhydride in den erfindungsgemäßen Zubereitungen eingesetzt werden.

Die Lösungsmittel der Zubereitungen sollen die Komponenten lösen und sollen, falls die Zubereitungen für dentale Zwecke bestimmt sind, untoxisch sein. Bevorzugt werden Wasser und flüchtige organische Lösungsmittel, wie Methanol, Ethanol, Propanol, Isopropanol, Aceton, Methylethylketon, Essigsäuremethyl- und -ethylester und Tetrahydrofuran. Im allgemeinen setzt man 10 bis 1000 Gew.-Teile, bevorzugt 50 bis 300 Gew.-Teile, des Lösungsmittels, bezogen auf die polymerisierbaren aromatischen Carbonsäuren und Carbonsäureanhydride ein. Besonders bevorzugt können auch Mischungen dieser Lösungsmittel sein, wobei wäßrige Mischungen ganz besonders bevorzugt sind.

Initiatoren im Rahmen der vorliegenden Erfindung sind Radikalbildner, die eine radikalische Polymerisation auslösen. Bevorzugt sind Photoinitiatoren, die unter der Einwirkung von Licht, beispielsweise UV-Licht, sichtbarem Licht oder Laserlicht, eine radikalische Polymerisation auslösen.

Die sogenannten Photopolymerisationsinitiatoren sind an sich aus der Literatur bekannt. Vorzugsweise handelt es sich um Mono- oder Dicarbonylverindungen, wie Benzophenon, Benzoin und dessen Derivate, insbesondere Benzoinmethylether, Benzil und Benzilderivate und andere Dicarbonylverbindungen, wie Diacetyl, 2,3-Pentandion und α-Diketoderivate des Norbornans und substituierter Norbornane, Metallcarbonyle, wie Pentacarbonylmangan, oder Chinone, wie 9,10-Phenanthrenchinon und Naphthochinon. Besonders bevorzugt ist Campherchinon.

Die erfindungsgemäßen Zubereitungen enthalten im allgemeinen 0,01 bis 2 Gew.-%, bevorzugt 0,1 bis 0,5 Gew.-%, des Initiators, bezogen auf die Menge an polymerisierbaren Verbindungen.

Enthält eines der mit der erfindungsgemäßen Zubereitung in Kontakt stehenden Fügeteile bereits einen Initiator der beschriebenen Art, so kann auf den Initiator in der Zubereitung verzichtet werden.

Es kann vorteilhaft sein, den erfindungsgemäßen Zubereitungen Coaktivatoren zuzusetzen, die die Polymerisationsreaktion beschleunigen. Bekannte Beschleuniger sind beispielsweise Amine, wie p-Toluidin und Dimethyl-p-toluidin, Trialkylamine, wie Trihexylamin, Polyamine, wie N,N,N',N'-Tetraalkylalkylendiamine, Barbitursäure und Dialkylbarbitursäuren. Besonders bevorzugt sind Dimethylaminobenzolsulfonamide entsprechend DE-A-31 35 113.

Die Coaktivatoren werden im allgemeinen in einer Menge von 0,02 bis 4 Gew.-%, bevorzugt 0,2 bis 1 Gew.-%, bezogen auf die Menge an polymerisierbaren Verbindungen, eingesetzt.

Als weitere Komponenten für die erfindungsgemäßen Zubereitungen kommen (Meth)acrylsäureester als Comonomere in Frage. Bevorzugt seien Ester der (Meth)acrylsäure mit 1- bis 5-wertigen Alkoholen mit 2 bis 30 Kohlenstoffatomen genannt. Besonders bevorzugt werden Epoxid(meth)acrylate und Urethan(meth)acrylate.

Außerdem seien Derivate des Tricyclodecans (EP-A-0 023 686) und Umsetzungsprodukte aus Polyolen, Diisocyanaten und Hydroxyalkylmethacrylaten (DE-A-37 03 120, DE-A-37 03 080 und DE-A-37 03 130) genannt.

Besonders bevorzugt als (Meth)acrylsäureester wird das sogenannte Bis-GMA der Formel

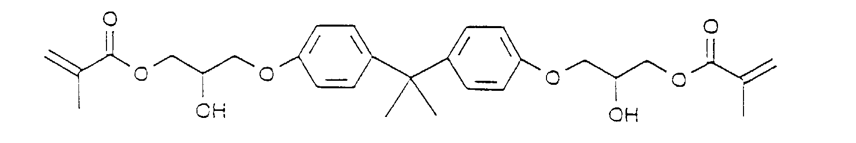

Es ist möglich, Mischungen der verschiedenen (Meth)acrylsäureester einzusetzen, beispielsweise Mischungen aus 20 bis 70 Gew.-Teilen Bis-GMA und 30 bis 80 Gew.-Teilen Triethylenglykoldimethacrylat.

Die erfindungsgemäßen Zubereitungen können weiterhin bis zu 10 Gew.-Teilen üblicher Zusätze, wie Stabilisatoren, Inhibitoren und Lichtschutzmittel, enthalten.

Die erfindungsgemäßen Zubereitungen können hergestellt werden, indem man die polymerisierbaren aromatischen Carbonsäuren und Carbonsäureanhydride, das Lösungsmittel, den Initiator und gegebenenfalls die weiteren Komponenten durch kräftiges Rühren miteinander mischt.

Die erfindungsgemäßen Zubereitungen werden vorzugsweise als Adhäsive, besonders zur Verbesserung der Haftung von polymerisierbaren Dentalmaterialien an der Zahnhartsubstanz - Schmelz und kollagenhaltiges Dentin -, verwendet.

In einer besonderen Ausführungsform konditioniert man die kollagenhaltige Zahnsubstanz vor der Behandlung mit den erfindungsgemäßen Zubereitungen mit einer Flüssigkeit, die einen pH-Wert im Bereich von 0,1 bis 3,5 aufweist. Diese Konditionierungsflüssigkeit enthält im allgemeinen Säuren mit einem pKₛ-Wert kleiner als 5 und gegebenenfalls eine amphotere Aminoverbindung mit einem pKₛ-Wert im Bereich von 9,0 bis 10,6 und einem pK_{b}-Wert im Bereich von 11,5 bis 12,5. Folgende Säuren können z. B. in der Konditionierungsflüssigkeit enthalten sein: Phosphorsäure. Salpetersäure, Brenztraubensäure, Zitronensäure, Oxalsäure, Ethylendiamintetraessigsäure, Essigsäure, Weinsäure, Äpfelsäure. Weiterhin kann die Konditionierungsflüssigkeit Stoffe aus der Gruppe der Polyethylenglykole und Metallhydroxide enthalten. Insbesondere können die oben aufgeführten mehrbasigen Säuren auch als teilweise Metallsalze einsetzt werden, solange freie Säurefunktionen verbleiben.

Die Anwendung der erfindungsgemäßen Zubereitungen als Adhäsive kann beispielsweise wie folgt durchgeführt werden:

Bei einer Zahnreparatur trägt man nach einer mechanischen Reinigung der kollagenhaltigen Zahnsubstanz zuerst die Konditionierungsflüssigkeit mit etwas Watte auf, läßt eine kurze Zeit (beispielsweise 60 Sekunden) einwirken, spült das Zahnmaterial mit Wasser und trocknet es im Luftstrom. Dann trägt man die erfindungsgemäße Zubereitung, beispielsweise mit einem kleinen Pinsel, in einer dünnen Schicht auf und trocknet im Luftstrom. Anschließend trägt man das eigentliche Füllungsmaterial, beispielsweise ein im Dentalbereich übliches Kunststoff-Füllungsmaterial, auf.

Neben ihrem Einsatz in als Adhäsive geeigneten Zubereitungen können die erfindungsgemäßen polymerisierbaren Carbonsäuren und Carbonsäureanhydride mit besonderem Vorteil auch in Anmischflüssigkeiten für Glasionomerzemente und in Knochenzementen verwendet werden.

### Experimenteller Teil

### Beispiele 1 bis 3

### Herstellung erfindungsgemäßer polymerisierbarer Carbonsäuren und Carbonsäuranhydride

### Beispiel 1

### Herstellung der Verbindungen 1 und 2 aus Tabelle 1

In 500 ml trockenem Butanon werden 160,2 g Glycerinmonomethacrylat (1 mol; Polyscience, Inc.) und 202 g (1 mol) Triethylamin gelöst. Der Ansatz wird auf -5° C gekühlt und ein Zulauf, bestehend aus 210,57 g Trimellitsäureanhydridchlorid und 180,6 g (1mol) Chlorameisensäureester der Formel (II), gelöst in 400 ml trockenem Butanon, tropfenweise zugegeben. Nach beendeter Zugabe wird 16 h bei 0° C gerührt. Dann wird der entstandene Niederschlag kalt abfiltriert, das Filtrat mit 0,1 n Salzsäure und mit Wasser ausgeschüttelt, die organische Phase abgetrennt und über Natriumsulfat getrocknet.

Die erhaltene Butanon-Lösung enthält die Verbindung 2 aus Tabelle 1 und kann direkt zur Hydrolyse der verbliebenen Anhydridgruppen eingesetzt werden. Dazu wird die Lösung mit 50 ml entionisiertem Wasser versetzt und über einen Zeitraum von 16 h bei Raumtemperatur gerührt. Nach Zusatz von 200 mg 2,6-Di-tert.-butylkresol läßt sich die erhaltene Lösung zu 372,1 g (75 % d. Th.) eines gelblichen viskosen Öls einengen (Verbindung 1 aus Tabelle 1)
IR: = 3400, 3200, 2950, 2600, 2400, 1800, 1735, 1640, 1490, 1460, 1390, 1290, 1250, 1185, 1100, 1060, 960, 870, 787, 715 cm⁻¹.
¹H-NMR (CDCl₃, 200 MHz): = 8.6 - 7,8 (3H); 6,2 und 5,7 (je 1 H); 5,2 (1 H); 4,8 - 4,3 (9H); 1,9 (3H) ppm.

### Beispiel 2

### Herstellung der Verbindung 6 aus Tabelle

In 500 ml trockenem Butanon werden 160,2 g Glycerinmonomethacrylat (1 mol; Polyscience, Inc.) und 202 g (1 mol) Triethylamin gelöst. Der Ansatz wird bei -5° C gekühlt und ein Zulauf, bestehend aus 327,18 g (1,5 mol) Pyromellitsäuredianhydrid und 180,6 g (1 mol) Chlorameisensäureester der Formel (II), gelöst in 400 ml trockenem Butanon, tropfenweise zugegeben. Nach beendeter Zugabe wird noch 2 h bei 0° C, dann 14 h bei Raumtemperatur gerührt. Anschließend wird der entstandene Niederschlag abfiltriert, das Filtrat mit 0,1 n Salzsäure und dann mit Wasser ausgeschüttelt, die organische Phase abgetrennt und über Natriumsulfat getrocknet.

Die erhaltene Butanon-Lösung enthält die gewünschte Verbindung 6 aus Tabelle 1. Nach Zusatz von 200 mg 2,6-Di-tert.-butylkresol läßt sich die Lösung zu 292,5 g (45 % d. Th.) eines gelblichen viskosen Öls (Verbindung 5 aus Tabelle 1) einengen.
IR: = 3400, 3050, 2950, 2550, 2350, 1800, 1720, 1640, 1550, 1495, 1460, 1390, 1350, 1250, 1170, 1115, 1060, 950, 760, 695 cm⁻¹.
¹H-NMR (Aceton-d₆, 200 MHz): = 8,3 - 8,0 (2H); 6,1 und 5,65 (je 1H); 5,1 (1H); 4,8 (1H) 4,3 - 4,2 (9H); 1,9 (3H) ppm.

### Beispiel 3

### Herstellung der Verbindung 15 aus Tabelle 1

In 500 ml trockenem Butanon werden 160,2 g Glycerinmonomethacrylat (1 mol; Polyscience, Inc.) und 202 g (1 mol) Triethylamin gelöst. Der Ansatz wird auf -5° C gekühlt und ein Zulauf, bestehend aus 402,27 g (1,5 mol) Naphthalin-1,4,5,8-tetracarbonsäuredianhydrid und 180,6 g (1 mol) Chlorameisensäureester der Formel (II), gelöst in 400 ml trockenem Butanon, tropfenweise zugegeben. Nach beendeter Zugabe wird noch 2 h bei 0° C, dann 14 h bei Raumtemperatur gerührt. Anschließend wird der entstandene Niederschlag abfiltriert, das Filtrat mit 0,1 n Salzsäure und dann mit Wasser ausgeschüttelt, die organische Phase abgetrennt und über Natriumsulfat getrocknet.

Die erhaltene Butanon-Lösung enthält die gewünschte Verbindung 15 aus Tabelle 1. Nach Zusatz von 200 mg 2,6-Di-tert.-butylkresol läßt sich die erhaltene Lösung auf 253,6 g (35 % d. Th.) eines gelblichen viskosen Öls einengen.
IR: = 3400, 3050, 2925, 2500, 2300, 1940, 1800 -1700, 1640, 1600, 1490, 1440, 1390, 1300, 1155, 1050, 955, 890, 820, 765, 720 cm⁻¹.

### Beispiel 4

### Prüfung der Photoreaktivität der (Meth)acryloyloxyalkylester mit Hilfe der Photo-DSC-Methode (Differential Scanning Calorimetry)

Die folgenden Bestandteile wurden intensiv miteinander gemischt:
- 5,0 g: (Meth)acryloyloxyalkylester
- 10 mg: Campherchinon
- 25 mg: p-Dimethylaminobenzolsulfonsäure-N,N-diallylamid (DASA).

Campherchinon und p-Dimethylaminobenzolsulfonsäure-N,N-diallylamid bilden das Photoinitiatorsystem.

Die Proben wurden bei 30° C in einer DSC-Apparatur mit einer Halogenlampe (75 W) mit Wärmeschutzfilter bestrahlt. Der Wärmefluß wurde unter Bestrahlung als Funktion der Zeit registriert. Als Referenz wurden Proben gleicher Zusammensetzung ohne Photoinitiator eingesetzt. Während des Versuchs wurde mit Stickstoff gespült. Für die Auswertung wurde als Maß für die Reaktionsgeschwindigkeit der Wert t-max ermittelt, t-max ist die Zeit von Bestrahlungsbeginn bis zum Erreichen des Maximums der Reaktion (maximaler Wärmefluß). Je kleiner t-max ist, um so größer ist die Photoreaktivität.

| (Meth)acryloyloxyalkylester | t-max [min] |
|---|---|
| Verbindung 1 aus Tabelle 1 | 0,63 |
| Verbindung 2 aus Tabelle 1 | 0,47 |
| 4-MET (Vergleich) | 1,10 |

### Beispiel 5

### Inhibition der Polymerisation durch Sauerstoff

Zur Prüfung der Inhibitionsempfindlichkeit der Monomeren wird die Dicke der nichtpolymerisierten Oberflächenschicht an Proben bestimmt, die gemäß Beispiel 4 mit Licht bestrahlt wurden.

Zylindrische Formen (6 mm Durchmesser, 0,5 mm Tiefe), die in eine rechteckige Messingplatte eingebohrt waren, werden mit dem zu untersuchenden Monomer in drei Schichten gefüllt und nach Abdampfen des Lösungsmittels 20 Sekunden lang mit dem Lichtgerät Translux CL (Heraeus Kulzer GmbH) unter Umgebungsatmosphäre bestrahlt und mit sehr wenig kolloidalem Silberpulver bestäubt. Die Messingplatte wird dann auf dem Tisch eines Auflichtmikroskopes gegen einen rechtwinkligen Rahmenhalter plaziert. Die Tischposition läßt sich mit Hilfe von zwei Stellmotoren in x- und in y-Richtung mit einer Reproduzierbarkeit von± 1 µm einstellen. Bei konstanter y-Stellung werden dann im Abstand von 1 mm entlang der x-Achse die Höhenkoordinaten z nach der Tiefenschärfemethode bestimmt. Die z-Wert-Erfassung erfolgt über einen senkrecht zur Tischebene am Tisch angebrachten Wegaufnehmer, der über ein kalibriertes Voltmeter die Höhenstellung in Mikrometereinheiten angibt. Die Reproduzierbarkeit der z-Wert-Bestimmung beträgt ± 1 µm. Unmittelbar nach der Ausgangswert-Bestimmung wird die Probenoberfläche mit Ethanol sorgfältig abgewaschen. Die Form wird dann auf den Mikroskoptisch zurückgebracht, und nach Anfahren der x/y-Ausgangspositionen werden erneut z-Werte bestimmt. Die Differenzen zwischen den 1. und 2. Messungen werden als Mittelwert pro Probe festgehalten und entsprechen der aufgrund der Inhibition durch Sauerstoff nichtpolymerisierten Oberflächenschicht. Pro Monomer werden drei Proben hergestellt und vermessen.

Je geringer die Dicke der nichtpolymerisierten Oberflächenschicht (Inhibitionsschicht) ist, desto geringer ist die Sauerstoff-Inhibition, desto besser ist die Aushärtung und damit die mechanische Festigkeit des Polymerisats und des zu klebenden Gesamtsystems.

### Ergebnisse

Unpolymerisierte Oberflächenschicht (µm)
- Verbindung 2 aus Tabelle 1: 1,3 ± 0,8
- 4-MET (Vergleichsversuch): gänzlich abwaschbar,
d. h. keine Aushärtung.

Nur die erfindungsgemäße Verbindung zeigt eine Aushärtung mit einer sehr geringen Inhibitionsschicht.

### Beispiele 6 und 7

### Herstellung von Zubereitungen zur Verwendung als Adhäsiv

### Beispiel 6

Eine Zubereitung gemäß der Erfindung zur Verwendung als Adhäsiv wird durch intensives Mischen der in diesem Beispiel aufgeführten Bestandteile erzeugt.
- 5 g: Aceton
- 2,5 g: Verbindung 1 aus Tabelle 1
- 2,5 g: Hydroxyethylmethacrylat
- 0,01 g: Campherchinon
- 0,025 g: DASA

### Beispiel 7

Zum Vergleich wird eine 4-MET enthaltende Zubereitung zur Verwendung als Adhäsiv durch intensives Mischen von
- 5 g: Aceton
- 5 g: 4-MET
- 0,01 g: Campherchinon
- 0,025 g: DASA
erzeugt.

Die Wirksamkeit der Adhäsive wurde geprüft durch Bestimmung der Scherbindungsfestigkeiten auf Schmelz und Dentin und durch mikroskopische Randanalyse an zylindrischen Dentinkavitäten, die mit einem konventionellen Komposit-Füllungsmaterial (Pekafill, Bayer) nach Konditionierung des Dentins und Aufbringen des Adhäsivs gefüllt waren. Es wurden menschliche Zähne benutzt, die für max. drei Monate nach der Extraktion in 1 % Chloraminlösung aufbewahrt waren. Vor der Verwendung im Test wurden die Zähne nach sorgfältiger Reinigung unter fließendem Wasser für mindestens drei und höchstens zehn Tage in physiologischer Kochsalzlösung gelagert.

### Scherbindungsfestigkeit

Am Tage vor der Verwendung im Bindungstest werden die Zähne einzeln auf einer Approximalseite liegend mit Epoxidharz (Lekutherm X20, Härter T3) in zylindrische Gummiformen von 25 mm Durchmesser und 12 mm Höhe eingebettet. Die Zähne werden durch Naßschleifen mit SiC-Papieren der Körnungen 240, 320, 400 und schließlich 600 soweit beschliffen, daß eine ausreichend große Schmelzoberfläche oder eine periphere Dentinfläche zur Anbindung eines Kunststoffzylinders mit 3,5 mm Durchmesser freiliegt. Nach Abspülen mit entionisiertem Wasser und Trocknen im Luftstrom wird das Gluma CPS Konditioniergel (20 % H₃PO₄) aufgetragen und nach 30 Sekunden mit Wasserspray sorgfältig abgespült. Die konditionierte Zahnoberfläche wird danach nur sehr kurz einem schwachen Luftstrom ausgesetzt, um das Wasser von der Oberfläche zu entfernen (feuchte Technik!). Das Adhäsiv wird mit einem Pinsel dünn aufgetragen, und durch vorsichtiges Abblasen mit Druckluft wird das Lösungsmittel abgedampft. Das Auftragen und Abdampfen wird zweimal wiederholt, bevor 20 Sekunden mit dem Lichtgerät Translux CL bestrahlt wird. Die vorbehandelte Probe wird dann mittels einer Einspannvorrichtung unter einer zweigeteilten zylindrischen Teflonform (3,5 mm Durchmesser, 1 mm Höhe) festgeklemmt. Das Füllungsmaterial wird aus einer Spritze appliziert, die mit Überschuß gefüllte Form mit einem lichtdurchlässigen Strip abgedeckt und schließlich 60 Sekunden lang mit dem Lichtgerät Translux CL bestrahlt. Unmittelbar anschließend wird die Teflonform abgenommen und die Probe in Wasser bei 37° C für 24 Stunden vor Einleitung einer Scherbelastung gelagert. Dazu wird die zylindrische Probe in einer Universalprüfmaschine mit Hilfe eines Druckstempels parallel zu und sehr dicht an der angeschliffenen Zahnoberfläche bei einer Geschwindigkeit von 1 mm/Minute bis zur Trennung des Zylinders vom Zahn belastet. Die Scherbindungsfestigkeit entspricht dem Quotienten aus Bruchkraft und Bindungsareal und wird in MPa angegeben. Die Lokalisierung der Fraktur wird im Stereomikroskop (Vergrößerung 60x) untersucht und als adhäsives oder kohäsives Versagen gekennzeichnet.

### Ergebnisse

Scherbindungsfestigkeit an Dentin (MPa)

| | |
|---|---|
| Zubereitung nach Beispiel 6 | 11,7 ± 0.4 |
| Zubereitung nach Beispiel 7 | 7,2 ± 0,9 |

### (Vergleichsversuch)

Nur bei erfindungsgemäßer Zubereitung aus Beispiel 6 lag die Fraktur grenzflächennah im Kunststoff (kohäsives Versagen). Die Zubereitung aus Beispiel 7 (Vergleichsversuch) zeigte adhäsives Versagen.

### Scherbindungsfestigkeit an Schmelz (MPa)

| | |
|---|---|
| Zubereitung nach Beispiel 6 | 20.1 ± 1.5 |
| Zubereitung nach Beispiel 7 | 8,3 ± 2,4 |

### (Vergleichsversuch)

Nur bei erfindungsgemäßer Zubereitung aus Beispiel 6 lag die Fraktur tief im Schmelz. Die Zubereitung aus Beispiel 7 (Vergleichsversuch) zeigte adhäsives Versagen.

Damit bestätigt sich, daß nur im Falle der Verwendung der erfindungsgemäßen Zubereitung die Bindung zwischen den Substraten stärker ist als die kohäsive Festigkeit von Kunststoff und Schmelz. Dies bestätigt die gute Leistungsfähigkeit der erfindungsgemäßen Zubereitungen.

### Mikroskopische Randanalyse

Zur Bestimmung der Kavitätenrandadaptation werden extrahierte Prämolaren oder Molaren approximal mit SiC-Papier der Körnung 600 naß angeschliffen, bis eine ausreichend große Dentinoberfläche exponiert ist, in die eine zylindrische Kavität (3 mm Durchmesser, ca. 1,5 mm Tiefe) präpariert werden kann. Die Kavität wird mit einem mittelkörnigen Diamantinstrument mit Hilfe eines schnell laufenden zahnärztlichen Winkelstücks unter Wasserkühlung ausgeformt. Nach sorgfältigem Reinigen mit Wasser erfolgt das Konditionieren und das Auftragen des Adhäsivs wie oben beschrieben, bevor das Komposit-Material Pekafill eingebracht, mit einem Strip abgedeckt und 60 Sekunden lang mit dem Lichtgerät Translux CL bestrahlt wird. Unmittelbar nach der Polymerisation werden die Zähne für 10 - 15 Minuten in Wasser bei Raumtemperatur gelagert, bevor der Überschuß an Füllungsmaterial durch vorsichtiges Naßschleifen mit SiC-Papier 600 und 4000 entfernt und der Kavitätenrand freigelegt wird. Anschließend wird im Auflichtmikroskop bei 500-facher Vergrößerung der Kavitätenrand inspiziert. Falls eine Ablösung des Füllungsmaterials erfolgt ist, wird die maximale Spaltbreite mit Hilfe eines Okularschraubenmikrometers bestimmt und in um angegeben. Die mikroskopische Inspektion wird innerhalb von maximal fünf Minuten durchgeführt, bevor als Folge des Trocknens Spalten auftreten können.

### Ergebnisse

Die erfindungsgemäße Zubereitung aus Beispiel 6 stellt sich als ausgezeichnet wirksam heraus. Es wurde kein Randspalt gefunden, die Kavitätenrandadaptation war perfekt. Die Anbindung an Dentin ist über eine Hybridschichtbildung erfolgt, die entsprechend der vorausgegangenen Konditionierung eine Schichtdicke von 10 - 14 um aufweist. Der Vergleich aus Beispiel 7 zeigt hingegen eine Ablösung des Füllungsmaterials mit einem Randspalt von 7 um.

### Beispiel 8

### Herstellung von Anmischflüssigkeiten für Glasionomerzemente

Anmischflüssigkeiten für lichthärtende Glasionomerzemente wurden durch Mischen der folgenden Bestandteile hergestellt:

| Bestandteile | **A** | **B** | **C** | **D** |
|---|---|---|---|---|
| 1,3-Glycerindimethacrylat [%] | 40 | 35 | 35 | 45 |
| HEMA * [%] | 39 | 29 | 34 | 44 |
| Verbindung 2 aus Tabelle 1 [%] | 10 | 25 | 15 | - |
| Wasser[%] | 10 | 10 | 15 | 10 |
| Campherchinon [%] | 0,45 | 0,45 | 0,45 | 0,45 |
| DASA [%] | 0,45 | 0,45 | 0,45 | 0,45 |
| Jonol (Stabilisator) | 0,10 | 0,10 | 0,10 | 0,10 |

| | | | | |
|---|---|---|---|---|
| * Hydroxyethylmethacrylat | | | | |

Zusätzlich wurde eine pulverförmige Mischung aus
a) 47 % Glasionomer-Pulver aus
   - 35,9: Gew.-% SiO₂
   - 22,5: Gew.-% Al₂O₃
   - 9: Gew.-% Na₃AlF₆
   - 6,6: Gew.-% AlF₃
   - 5: Gew.-% AlPO₄
   - 21: Gew.-% CaF₂,
b) 47 % Barium-haltigem Dentalglas "Typ GM 27884" (Firma Schott), d₅₀ca. 1,3 um, und
c) 6 % Aerosil VP-R 711 (Firma Degussa)
hergestellt.

Drei Teile der pulverförmigen Mischung werden mit je einem Teil der Anmischflüssigkeiten A - D gemischt und mit dem Lichtgerät Translux CL 60 sec lang bestrahlt.

Durch die in Beispiel 5 beschriebene Methode zur Bestimmung der Inhibition wurde die Dicke der Inhibitionsschichten mit folgenden Werten ermittelt:

| Anmischflüssigkeit | Verbindung 2 aus Tabelle 1 [%] | Inhibitionsschicht [um] |
|---|---|---|
| A | 10 | 2,9 |
| B | 25 | 1,5 |
| C | 15 | 2,81 |
| D | - | 12,6 |

Nur die Anmischflüssigkeiten mit der erfindungsgemäßen Verbindung zeigen eine gute Lichthärtung mit signifikanter Verringerung der Dicke der Inhibitionsschicht.

## Patentansprüche

1. Polymerisierbare (Meth)Acrylsäureester von Derivaten aromatischer Carbonsäuren oder Carbonsäureanhydride mit cyclischen Carbonat-Gruppen der Formeln 1 bis 16.

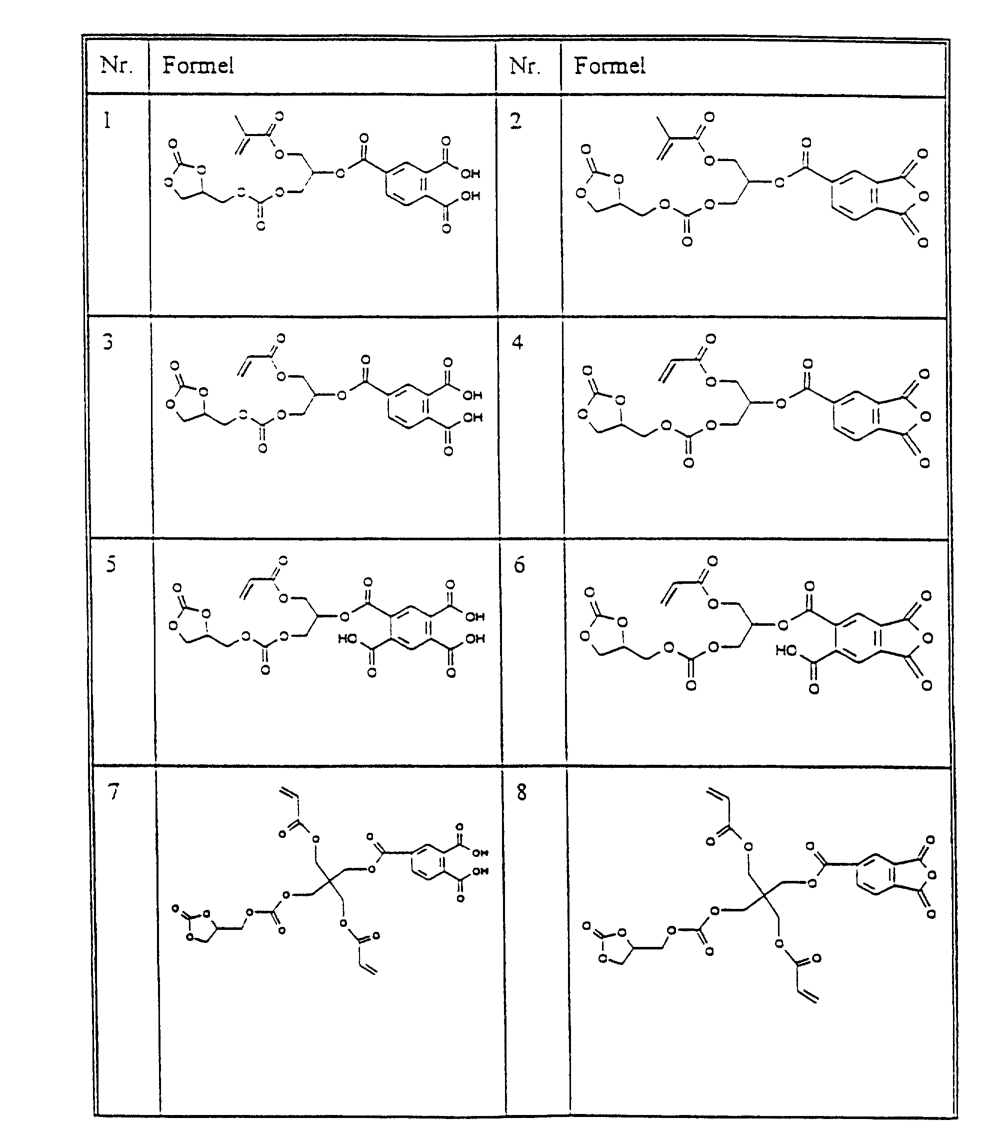

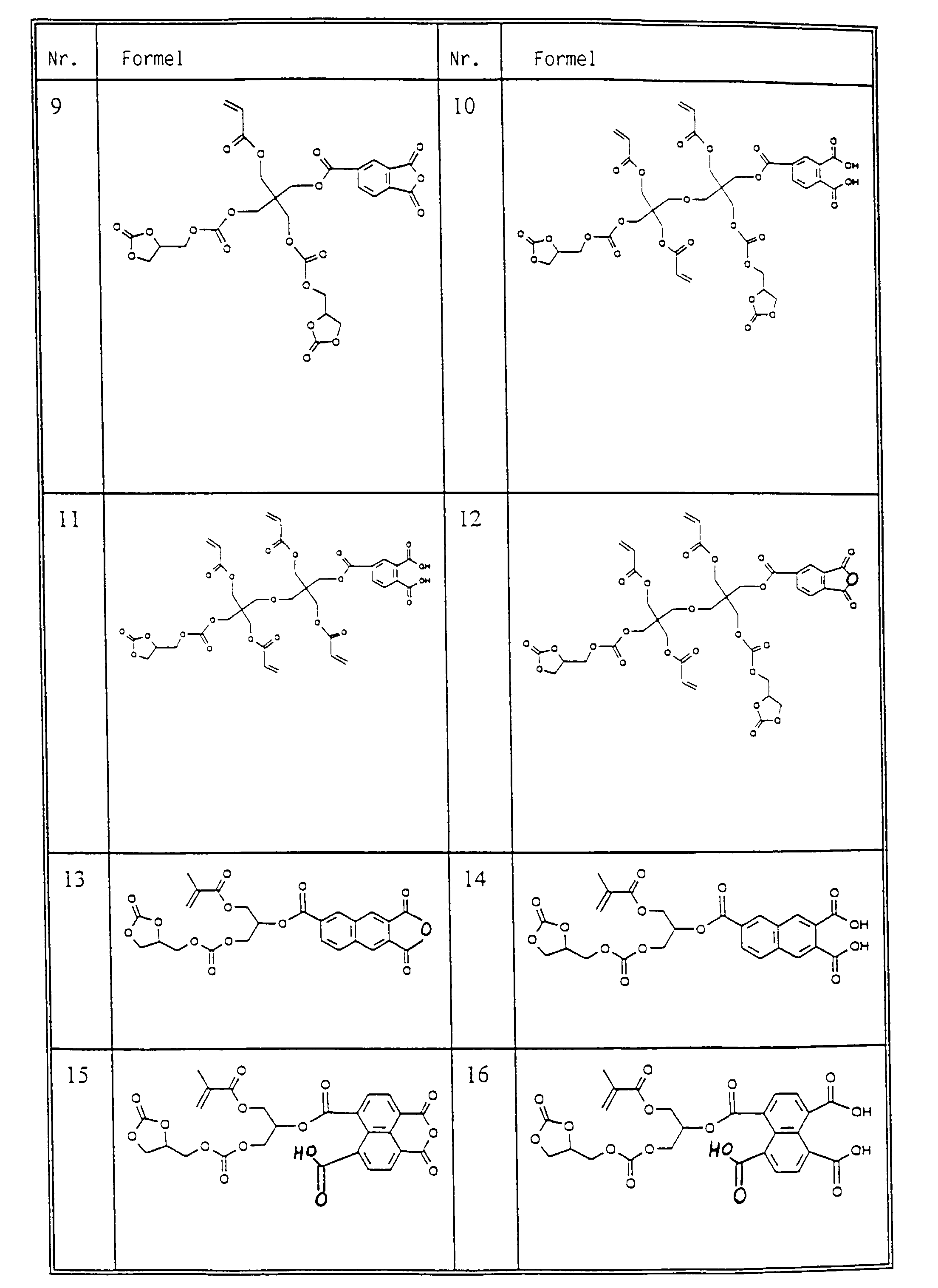

2. Zubereitungen, enthaltend polymerisierbare (Meth)Acrylsäureester gemäß Anspruch 1.

3. Zubereitungen nach Anspruch 2, dadurch gekennzeichnet, daß sie als Photopolymerisationsinitiator Radikalbildner aus der Reihe der Mono- oder Dicarbonylverbindungen enthalten.

4. Zubereitungen nach Anspruch 3, dadurch gekennzeichnet, daß der Photopolymerisationsinitiator Champherchinon ist.

5. Zubereitungen nach Anspruch 3 oder 4, dadurch gekennzeichnet, daß sie zusätzlich Coaktivatoren enthalten.

6. Zubereitungen nach Anspruch 5, dadurch gekennzeichnet, daß die Coaktivatoren Amine sind.

7. Zubereitungen nach Anspruch 5, dadurch gekennzeichnet, daß die Coaktivatoren Dimethylaminobenzolsulfonamide sind.

8. Verwendung der Zubereitungen gemäß Ansprüchen 2 bis 7 als Adhäsive.

9. Verwendung der Zubereitungen gemäß Ansprüchen 2 bis 7 in der Dentaltechnik.

10. Verwendung der Zubereitungen gemäß Ansprüchen 2 bis 7 als Anmischflüssigkeiten für Glasionomerzemente.

11. Verwendung der Zubereitungen gemäß Ansprüchen 2 bis 7 in Knochenzementen.

## Claims

1. Polymerizable (meth)acrylic esters of derivatives of aromatic carboxylic acids or carboxylic anhydrides having cyclic carbonate groups of the formulae 1 to 16.

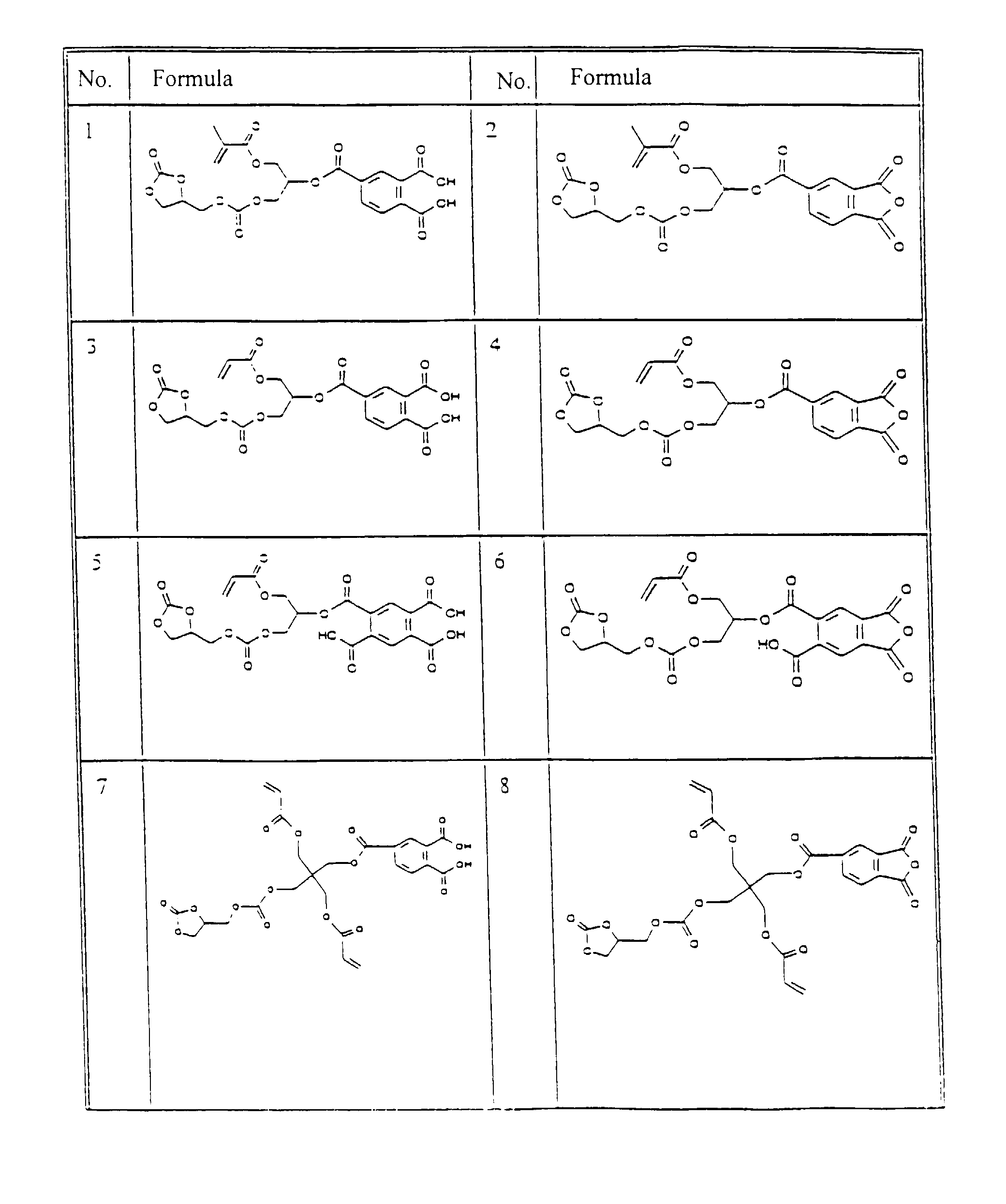

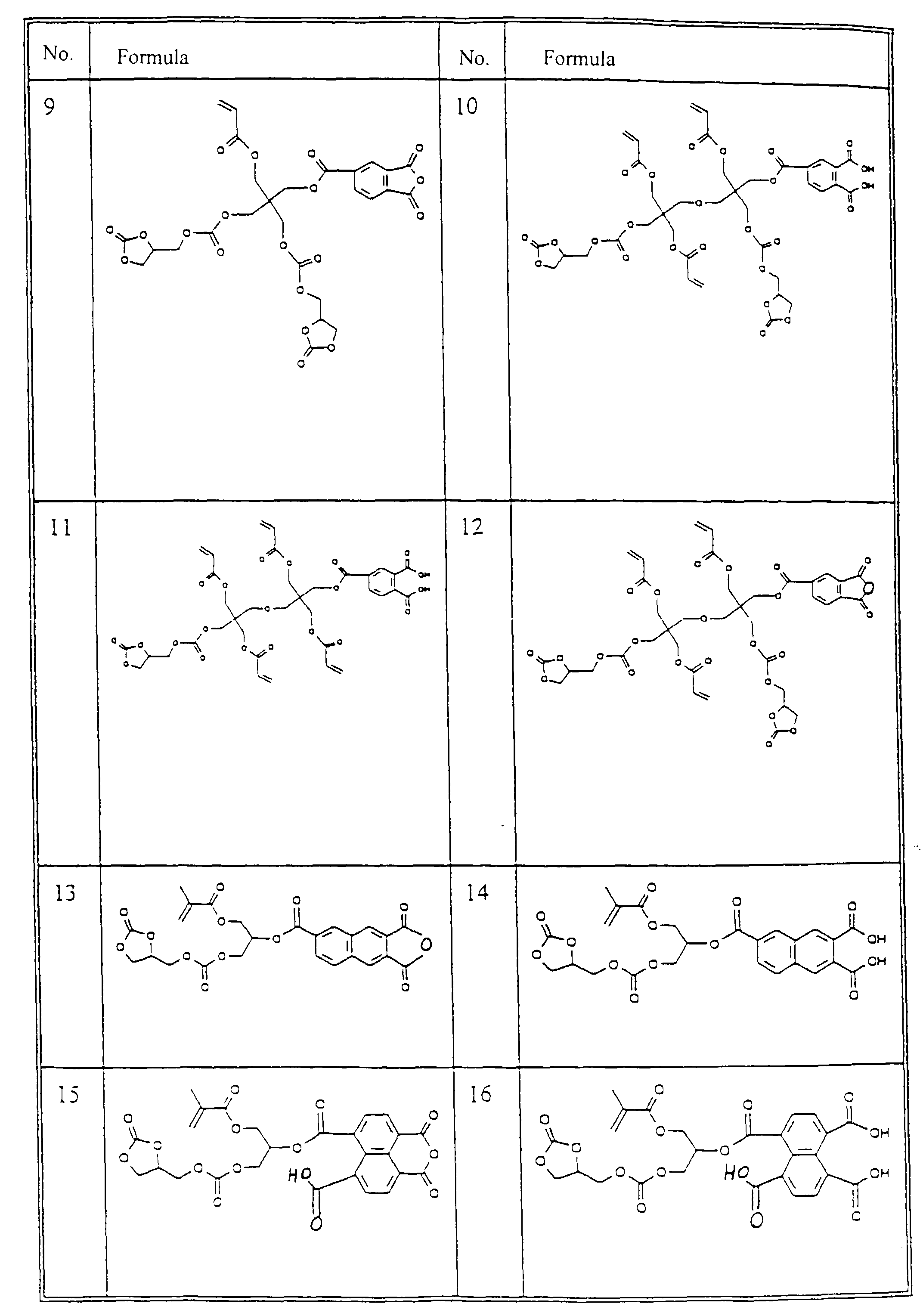

2. Formulations containing polymerizable (meth)acrylic esters according to Claim 1.

3. Formulations according to Claim 2, characterized in that they contain, as a photopolymerization initiator, free radical formers from the series consisting of the mono- or dicarbonyl compounds.

4. Formulations according to Claim 3, characterized in that the photopolymerization initiator is camphorquinone.

5. Formulations according to Claim 3 or 4, characterized in that they additionally contain coactivators.

6. Formulations according to Claim 5, characterized in that the coactivators are amines.

7. Formulations according to Claim 5, characterized in that the coactivators are dimethylaminobenzenesulphonamides.

8. Use of the formulations according to Claims 2 to 7 as adhesives.

9. Use of the formulations according to Claims 2 to 7 in dental technology.

10. Use of the formulations according to Claims 2 to 7 as mixing liquids for glass ionomer cements.

11. Use of the formulations according to Claims 2 to 7 in bone cements.

## Revendications

1. Esters polymérisables de l'acide (méth)acrylique de dérivés d'acides carboxyliques ou d'anhydrides d'acides carboxyliques aromatiques contenant des groupes carbonate cycliques de formules 1 à 16.

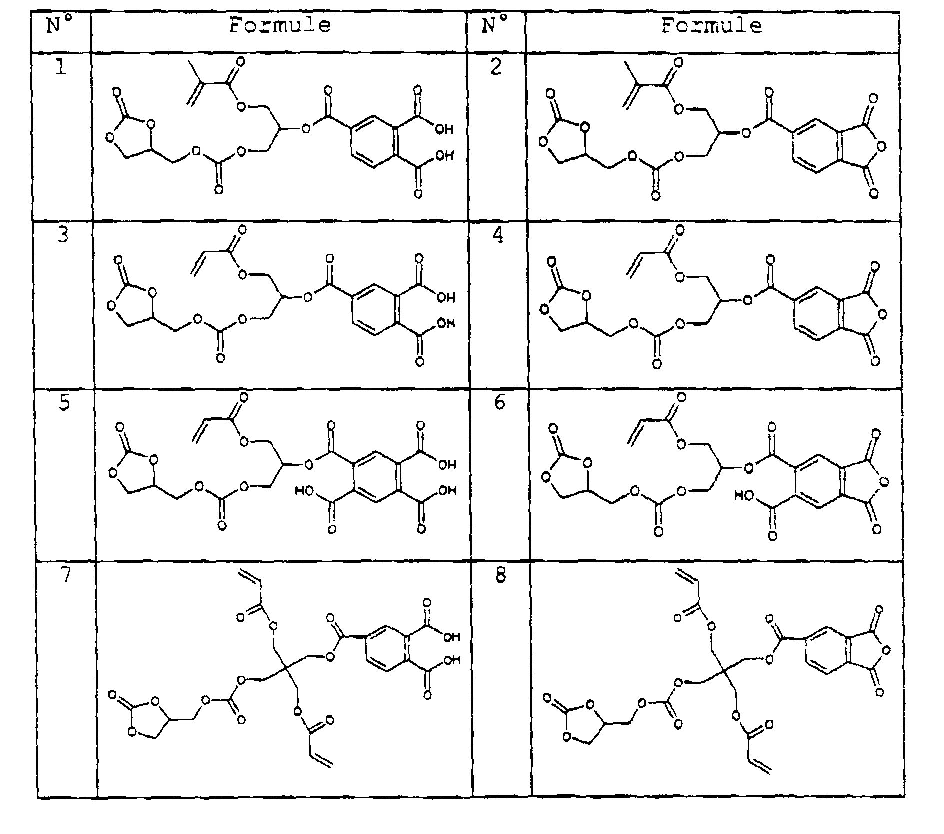

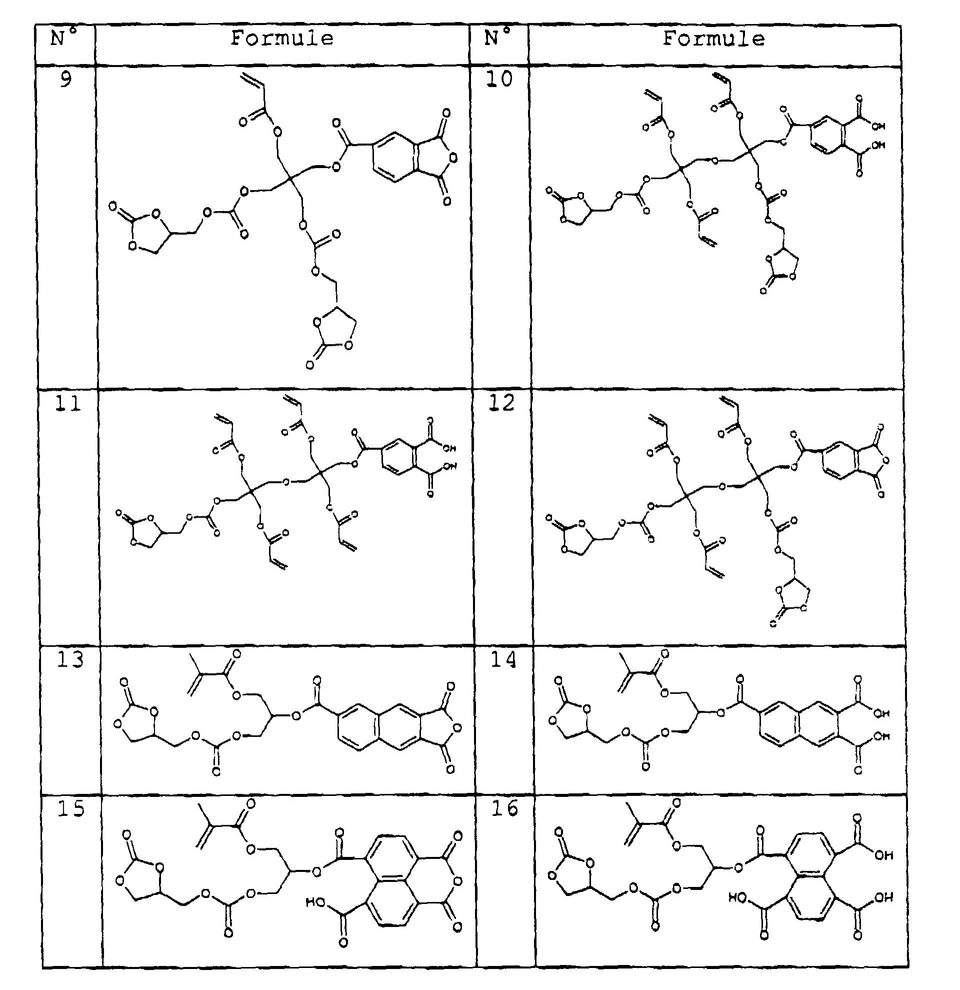

2. Préparations contenant des esters polymérisables de l'acide (méth)acrylique selon la revendication 1.

3. Préparations selon la revendication 2, caractérisées en ce qu'elles contiennent en tant qu'amorceur de photopolymérisation des agents formant des radicaux de la série des composés mono- ou dicarbonylés.

4. Préparations selon la revendication 3, caractérisées en ce que l'amorceur de photopolymérisation est la camphoquinone.

5. Préparations selon la revendication 3 ou 4, caractérisées en ce qu'elles contiennent en outre des co-activateurs.

6. Préparations selon la revendication 5, caractérisées en ce que les co-activateurs sont des amines.

7. Préparations selon la revendication 5, caractérisées en ce que les co-activateurs sont des diméthylaminobenzènesulfonamides.

8. Utilisation des préparations selon les revendications 2 à 7, en tant qu'adhésifs.

9. Utilisation des préparations selon les revendications 2 à 7, dans la technique dentaire.

10. Utilisation des préparations selon les revendications 2 à 7, en tant que liquides de trituration pour ciments ionomères pour le verre.

11. Utilisation des préparations selon les revendications 2 à 7, dans des ciments osseux.
